(19) Europäisches Patentamt
European Patent Office
Office européen des brevets

(11) **EP 2 348 984 B1**

(12) **EUROPÄISCHE PATENTSCHRIFT**

(45) Veröffentlichungstag und Bekanntmachung des Hinweises auf die Patenterteilung:
**29.04.2020 Patentblatt 2020/18**

(21) Anmeldenummer: **09755837.3**

(22) Anmeldetag: **14.10.2009**

(51) Int Cl.:
***A61B 5/053*** (2006.01)

(86) Internationale Anmeldenummer:
**PCT/EP2009/007366**

(87) Internationale Veröffentlichungsnummer:
**WO 2010/043381 (22.04.2010 Gazette 2010/16)**

(54) **VERFAHREN ZUR BESTIMMUNG EINES KORRIGIERTEN VOLUMENKOMPARTIMENTS EINES AMPUTIERTEN PATIENTEN, VORRICHTUNG ZUR DURCHFÜHRUNG DES VERFAHRENS UND COMPUTERPROGRAMMPRODUKT**

METHOD FOR DETERMINING A CORRECTED VOLUME COMPARTMENT OF AN AMPUTATED PATIENT, DEVICE FOR CARRYING OUT THE METHOD AND COMPUTER PROGRAM PRODUCT

PROCÉDÉ POUR DÉTERMINER UN COMPARTIMENT CORPOREL CORRIGÉ CHEZ UN PATIENT AMPUTÉ, DISPOSITIF POUR EXÉCUTER CE PROCÉDÉ ET PRODUIT-PROGRAMME INFORMATIQUE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **15.10.2008 DE 102008051347**

(43) Veröffentlichungstag der Anmeldung:
**03.08.2011 Patentblatt 2011/31**

(73) Patentinhaber: Fresenius Medical Care
**Deutschland GmbH
61352 Bad Homburg (DE)**

(72) Erfinder:
• **MOISSL, Ulrich
61148 Karben (DE)**
• **WABEL, Peter
61191 Rosbach (DE)**
• **WIESKOTTEN, Sebastian
64560 Erfelden (DE)**

(74) Vertreter: **Dreyhsig, Jörg
Fresenius Medical Care AG & Co. KGaA
Global Intellectual Property
Frankfurter Strasse 6-8
66606 St. Wendel (DE)**

(56) Entgegenhaltungen:
**WO-A1-2006/002656**

• **ROTTMANN ET AL: "Bestimmung der Koerperzusammensetzung mittels Bioimpedanzspektroskopie unter Beruecksichtigung von Amputationen" DIPLOMARBEIT,, 14. Mai 2007 (2007-05-14), Seiten 1-69, XP009133889**
• **WIESKOTTEN S ET AL: "Konzeptentwurf fuer eine Modell-basierte Bestimmung von Koerperzusammensetzung und Ueberwaesserung bei Dialysepatienten mt Amputationen" AUTOMATISIERUNGSTECHNISCHE VERFAHREN FÜR DIE MEDIZIN : 7. WORKSHOP ; TAGUNGSBAND : 2007.10.19-20, VDI-VERLAG, DE, 1. Januar 2007 (2007-01-01), Seiten 21-22, XP009133376 ISBN: 978-3-18-326717-0**

**Beschreibung**

Die Erfindung betrifft das Gebiet zur Überwachung des Hydrations- und/oder Ernährungszustandes eines Patienten mit Hilfe von Bioimpedanzmesswerten.

Die Nieren haben mehrere Funktionen, um den Gesundheitszustand eines menschlichen Körpers aufrecht zu erhalten. Zunächst kontrollieren sie die Flüssigkeitsbilanz durch das Abtrennen jeglicher überflüssiger Flüssigkeit aus dem Blutvolumen des Patienten. Zweitens dienen sie dazu, das Blut von jeglichen Abfallstoffen wie Harnstoff oder Kreatinin zu reinigen. Nicht zuletzt kontrollieren sie auch den Spiegel von bestimmten Substanzen im Blut, wie Elektrolyten, um einen gesunden und notwendigen Konzentrationsspiegel zu gewährleisten.

Im Falle eines Nierenversagens reichert sich aufgenommene Flüssigkeit im Körpergewebe an und verursacht zunehmenden Stress auf den Blutkreislauf beziehungsweise das vaskuläre System. Dieses Übermaß an Flüssigkeit muss während einer Dialysebehandlung durch Ultrafiltration aus dem Blut entfernt werden. Wenn eine unzureichende Menge Flüssigkeit entfernt wird, können die langfristigen Konsequenzen schwerwiegend sein und zu hohem Blutdruck und Herzversagen führen. Die Wahrscheinlichkeit des Auftretens eines Herzversagens ist bei Dialysepatienten deutlich erhöht, und es wird angenommen, dass die Zustände einer Flüssigkeitsüberlastung einer der entscheidenden Faktoren ist. Ein Entfernen von zuviel Flüssigkeit ist ebenso gefährlich, da der Dialysepatient dehydriert wird und dies zu einer Hypotonie führt.

Das Trockengewicht (der Einfachheit halber sollen die Worte "Gewicht" und "Masse" im Rahmen dieser Patentanmeldung synonym verwendet werden - was der üblichen Praxis im medizinischen Gebiet entspricht) definiert das Gewicht eines Patienten, das erreicht werden würde, wenn die Nieren normal arbeiten. In anderen Worten, es repräsentiert das optimale Zielgewicht (oder den Flüssigkeitsstatus), das erreicht werden sollte, um das kardiovaskuläre Risiko zu minimieren. Das Trockengewicht war immer ein schwer fassbares Problem in der allgemeinen klinischen Praxis, da es an quantitativen Verfahren zu dessen Bestimmung fehlt. Zur Zeit wird das Trockengewichtproblem unter Verwendung indirekter Indikatoren wie z. B. Blutdruck, echokardiographischen Untersuchungen und subjektiven Informationen wie Röntgenaufnahmen angegangen. Darüber hinaus war es äußerst schwierig, eine Zusammenstellung von Bedingungen zu definieren, die allgemein als Trockengewichtsstandard akzeptiert werden.

Ein viel versprechendes Verfahren, den Flüssigkeitszustand eines Patienten herzuleiten, involviert die Verwendung von Bioimpedanzmessungen. Ein niedriger Wechselstrom wird über zwei oder mehrere Elektroden, die am Patienten angebracht sind, angelegt, und es wird die entsprechende elektrische Potentialdifferenz gemessen. Die verschiedenen Flüssigkeitskompartimente des menschlichen Körpers tragen auf verschiedene Art zu dem gemessenen Signal bei. Die Verwendung von multiplen Frequenzen erlaubt es, das intrazelluläre Wasservolumen (ICV) und das extrazelluläre Wasservolumen (ECV) zu bestimmen. Mit diesen Angaben ist eine Bestimmung des Hydrationszustandes im Sinne eines Überschusses an Flüssigkeit oder einer Dehydrierung möglich. Ein Beispiel für eine solche Vorrichtung ist in der internationalen Patentanmeldung WO 2006/002685 beschrieben. Mit einer derartigen Vorrichtung ist es gleichzeitig möglich, die Körperzusammensetzung bezüglich anderer Volumenkompartimente des Patienten zu bestimmen, insbesondere den Anteil des Mager- und des Fettgewebes. Auf diese Weise sind auch Aussagen über den Ernährungszustand des Patienten möglich.

Den für die Auswertung von Bioimpedanzmessungen zugrunde liegenden Modellen ist dabei gemeinsam, dass sie entweder von einem Patientenkörper ausgehen, dem keine Extremitäten oder Gliedmaßen fehlen, oder sich statt auf eine Ganzkörpermessung nur auf eine segmentale Messung an einem bestimmten Körpersegment beschränken.

In diesem Zusammenhang stellt sich die Frage, wie eine Ganzkörpermessung zur Bestimmung der Körperzusammensetzung bei amputierten Patienten durchgeführt werden kann, denen einzelne Gliedmaße oder Teile davon fehlen. Amputationen von Extremitäten sind in einer Dialysepopulation häufiger anzutreffen als in der Normalbevölkerung. Die Ursache hierfür ist meist Diabetes. Ein zu hoher Blutzuckerspiegel schädigt die feinen Kapillargeflechte des Körpers, insbesondere in den Nieren und den Extremitäten. Mögliche Folgen von Diabetes sind Nierenversagen und die Amputation von Extremitäten.

Mit Hilfe einer so genannten 4-Punkt Messung sind Versuche unternommen worden, die segmentalen Anteile des Körpers von gesunden Probanden zu ermitteln und auf dieser Basis den Einfluss von Amputationen zu untersuchen (Wieskotten et. al, in: Tagungsband Automatisierungstechnische Verfahren für die Medizin - 7. Workshop 2007, ISBN 978-3-18-326717-0). Rottmann et. al.: "Bestimmung der Körperzusammensetzung mittels Bioimpedanzspektroskopie unter Berücksichtigung von Amputationen; Diplomarbeit, 14. May 2007, Seiten 1 -69, XP009133889" beschreibt ein Verfahren zur Bestimmung der Körperzusammensetzung bei Amputierten.

Der Erfindung liegt die Aufgabe zugrunde, ein Verfahren bereitzustellen, mit der die Körperzusammensetzung eines amputierten Patienten mit Hilfe von Bioimpedanzmessungen einfach und individuell ermittelt werden kann. Der Erfindung liegt auch die Aufgabe zugrunde, eine Vorrichtung für eine solche Bioimpedanzmessung zur Bestimmung der Körperzusammensetzung eines amputierten Patienten zur Verfügung zu stellen. Die Erfindung betrifft auch ein Computerprogrammprodukt zur Durchführung des erfindungsgemäßen Verfahrens.

Nach der Lehre der Erfindung werden diese Aufgaben durch ein Verfahren mit den Merkmalen des Anspruches 1, durch eine Vorrichtung mit den Merkmalen des Anspruches 8 sowie durch ein Computerprogrammprodukt mit den Merkmalen des Anspruches 15 gelöst. Vorteilhafte Ausgestaltungen sind Gegenstand der Unteransprüche.

Die Erfindung beruht auf der Erkenntnis, dass es zur Berücksichtigung fehlender Gliedmaße oder Extremitäten zur Bestimmung der Körperzusammensetzung gar keiner angepassten Patientenmodelle bedarf, sondern vielmehr zunächst von dem ursprünglichen Körpermodell ausgegangen werden kann, dass für den Fall verwendet wird, dass einem Patienten keine Gliedmaßen oder Teile davon fehlen. Unter Körperzusammensetzung wird in diesem Sinne eine Teilkomponente des menschlichen Körpers verstanden, die als Volumenkompartiment angegeben werden kann, sei es in einer Volumen- oder einer Masseneinheit, absolut oder relativ.

Gemäß des erfindungsgemäßen Verfahrens kann ein korrigiertes Volumenkompartiment V(t) eines Patienten zum Zeitpunkt t, wobei dem Patienten einzelne Gliedmaßen oder Teile davon fehlen, danach bestimmt werden, dass zunächst Bioimpedanzdaten am Patienten gemessen werden, dann ein Volumenkompartiment V'(t) bestimmt wird und anschließend das korrigierte Volumenkompartiment V(t) durch Multiplikation des Volumenkompartiments V'(t) oder einer damit korrelierten Größe mit einem für das fehlende Gliedmaß oder Teile davon charakteristischen Faktor k erhalten wird.

Die Volumenkompartimente können zum Beispiel Magergewebe (LTM), Fettgewebe (ATM), Überwasserungsvolumen (OH), extrazelluläres Wasser (ECW), intrazelluläres Wasser (ICW) umfassen oder aus diesen Parametern ausgewählt sein.

In einer bevorzugten Ausführungsform wird die Bioimpedanzmessung als Ganzkörpermessung durchgeführt. Als Ganzkörpermessung wird in diesem Zusammenhang eine Messung verstanden, bei denen der elektrische Strom mehrere Körpersegmente durchdringt, die mindestens den Körperrumpf umfassen. Bei Fehlen eines Armes oder Teilen davon und/oder Fehlen eines Beines oder Teilen davon kann die Ganzkörpermessung mit Spannungs- und Stromelektroden durchgeführt wird, die in der Nähe eines vorhandenen Handgelenks und in der Nähe eines vorhandenen Fußgelenks angebracht werden. Bei Fehlen beider Beine oder Teilen davon können die Spannungs- und Stromelektroden in der Nähe beider Handgelenke, bei Fehlen beider Arme oder Teilen davon in der Nähe beider Fußgelenke angebracht werden.

In einer weiteren Ausführungsform wird der Korrekturfaktor k auf der Basis von Vergleichsdaten ermittelt, bei dem der Anteil des fehlenden Gliedmaßes oder eines Teiles davon aufgrund seines Anteils am Gesamtgewicht eines Vergleichspatienten ohne fehlende Gliedmaße oder Teile davon bestimmt wird.

Bei einer besonders einfachen Anwendung des erfindungsgemäßen Verfahrens wird das Körpergewicht M(t) des Patienten ermittelt und bei der Bestimmung des Volumenkompartiments V'(t) ein korrigiertes Körpergewicht M'(t) verwendet, dass sich aus dem Quotienten des gemessenen Körpergewichtes M(t) geteilt durch den Korrekturfaktor k ergibt. Auf diese Weise erfolgt die Auswertung der Messwerte, als ob sie an Patienten ohne amputierte Gliedmaße vorgenommen werden. Nach der Bestimmung des Volumenkompartiments V'(t) muss dem Umstand Rechnung getragen werden, dass das Volumenkompartiment um das nicht vorhandene Gliedmaß anteilig korrigiert werden muss, was erfindungsgemäß durch Multiplikation mit dem Korrekturfaktor k gelingt.

Die erfindungsgemäße Vorrichtung ist zur Durchführung des erfindungsgemäßen Verfahrens ausgebildet. Sie umfasst eine Bioimpedanzmesseinrichtung zur Messung von Bioimpedanzdaten am Patienten sowie eine Auswerteeinheit, die so konfiguriert ist, dass anhand der gemessenen Bioimpedanzdaten ein Volumenkompartiment V'(t) des Patienten bestimmt wird und das korrigierte Volumenkompartiment V(t) durch Multiplikation des Volumenkompartiments V'(t) oder einer damit korrelierten Größe mit einem für das fehlende Gliedmaß oder Teile davon charakteristischen Faktor k bestimmt wird.

Mit Hilfe des erfindungsgemäßen Computerprogrammproduktes kann eine Vorrichtung, die eine Bioimpedanzmesseinrichtung sowie eine programmierbare Auswerteeinheit umfasst, durch Übertragung des auf dem Computerprogrammprodukt gespeicherten Programms in die Auswerteeinheit erfindungsgemäß konfiguriert werden, wodurch die Ausführung des erfindungsgemäßen Verfahrens ermöglicht wird.

Weitere Einzelheiten und Vorteile der Erfindung werden anhand eines in den Zeichnungen dargestellten Ausführungsbeispiels näher beschrieben. Es zeigen:

Fig. 1 eine Ausführungsform einer Vorrichtung zur Bestimmung eines korrigierten Volumenkompartiments V(t) eines Patienten zum Zeitpunkt t, wobei dem Patienten einzelne Gliedmaße oder Teile davon fehlen, in schematischer Form,

Fig. 2 die Anordnung der Bioimpedanzelektroden zur Ganzkör-perbioimpedanzmessung bei fehlenden Gliedmaßen oder Extremitäten: (a) zum Vergleich ohne fehlende Gliedmaße, (b) bei einem fehlenden Arm, (c) bei einem fehlenden Bein und (d) bei einem fehlenden Arm und einem fehlenden Bein,

Fig. 3 die Anordnung der Bioimpedanzelektroden zur Ganzkörperbioimpedanzmessung bei fehlenden Gliedmaßen oder Extremitäten: (a) bei Fehlen beider Arme und (b) bei Fehlen beider Beine,

Fig. 4 eine Tabelle zur Ermittlung des Korrekturfaktors k und

Fig. 5 beispielhafte Daten für zusätzliche Parameterwerte für die Auswertung im Falle der in Fig. 3 gezeigten Anordnungen.

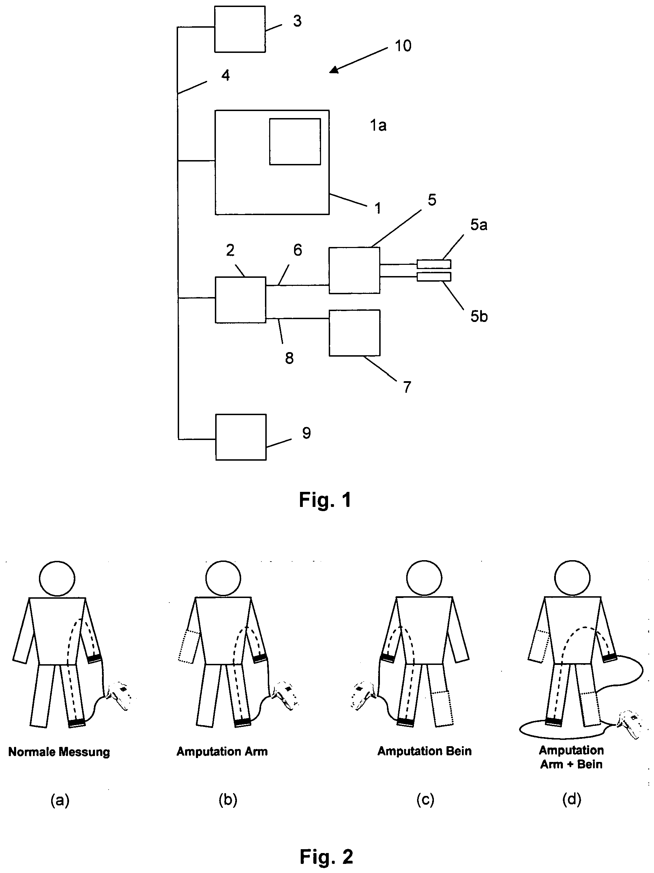

Fig.1 zeigt eine Ausführungsform der erfindungsgemäßen Vorrichtung 8. Eine solche Ausführungsform einer Vorrichtung zur Bestimmung eines korrigierten Volumenkompartiments V(t) eines Patienten, wobei dem Patienten einzelne Gliedmaßen oder Teile davon fehlen, umfasst eine als Mikroprozessoreinheit ausgebildete Auswerteeinheit 1, welche wiederum eine Mikroprozessorprogrammspeichereinheit 1a umfasst. Die Auswerteeinheit 1 ist über Verbindungsmittel 4 mit einer Eingangseinheit 2 und einer Speichereinheit 3 verbunden. Ein Programm zur Bestimmung eines korrigierten Volumenkompartiments V(t) eines Patienten zum Zeitpunkt t, wobei dem Patienten einzelne Gliedmaßen oder Teile davon fehlen, ist in der Mikroprozessorprogrammspeichereinheit 1a gespeichert.

Zur Bestimmung von Bioimpedanzmessdaten wird eine Bioimpedanzmesseinrichtung 5 bereitgestellt, welche über eine Verbindung 6 mit der Eingabeeinheit 2 verbunden ist. Für eine Bioimpedanzmessung sind verschiedene Elektrodenanordnungen möglich. In Fig. 1 sind nur 2 Elektrodenelemente 5a und 5b an die Bioimpedanzmesseinrichtung 5 angeschlossen. Jede der Elektrodeneinheiten 5a und 5b besteht aus einer Strom injizierenden Elektrode und einer Potential aufnehmenden Elektrode (nicht gezeigt). Durch das Anbringen der zwei Elektrodeneinheiten 5a und 5b an das Handgelenk beziehungsweise das Fußgelenk eines Patienten, wie es in Fig. 2(a) skizziert ist, kann die Ganzkörperimpedanz eines Patienten in üblicher Weise bestimmt werden, wenn dem Patienten keine Gliedmaße oder Extremitäten fehlen.

Es können ferner Mittel 7 zur Bestimmung der Größe H(t) des Patienten vorhanden sein, wobei die Mittel über eine Verbindung 8 mit der Eingabeeinheit 2 verbunden sind. Die Mittel 7 bestehen aus einer Messvorrichtung, wie sie im Stand der Technik bekannt sind. In einem weiterentwickelten Ausführungsbeispiel der Erfindung umfassen die Mittel 7 auch Wiegemittel zur Bestimmung des Gewichts M(t) des Patienten.

In dem in Fig.1 gezeigten Ausführungsbeispiel beinhaltet die Eingabeeinheit 2 ein Interface, über welches die Bioimpedanzmesswerte sowie die Werte für H(t) und M(t) direkt über eine Verbindung 4 in die Speichereinheit 3 transferiert werden. Es ist ebenfalls möglich, dass die Werte oder ein Teil davon manuell durch einen Benutzer in die Eingabeeinheit 2 eingegeben werden.

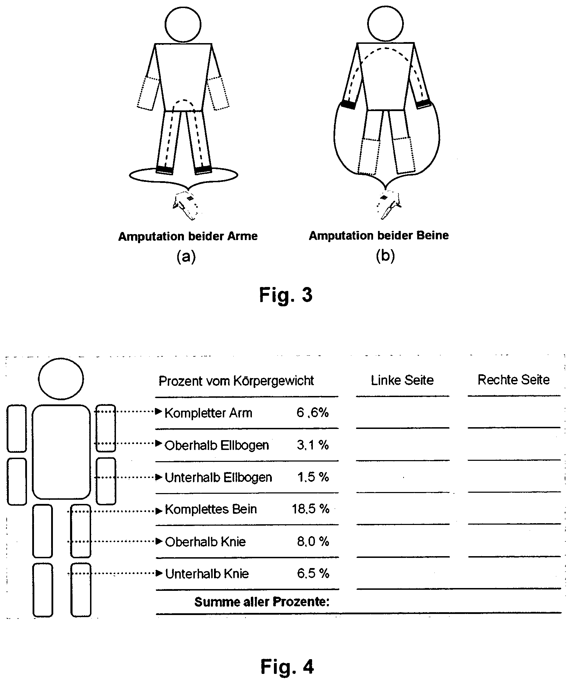

In der Speichereinheit 3 sind ferner Korrekturfaktoren k entsprechend verschiedener Amputationsgrade hinterlegt. Diese Faktoren sind auf der Basis von Vergleichsdaten, die für Patienten gewonnen wurden, denen die Gliedmaße oder Teile davon nicht fehlen, zuvor ermittelt worden. Sie können auch ein integraler Bestandteil des in der Mikroprozessorprogrammspeichereinheit 1a hinterlegten Computerprogramms sein. Die Korrekturfaktoren können aus Bioimpedanz- oder Gewichtsvergleichsmessungen gewonnen worden sein und zum Beispiel einfach in Form von prozentualen Gewichtsanteilen vorliegen. Ein solches Beispiel ist in Fig. 4 für einzelne Extremitäten wie kompletten Armen und Beinen als auch Teilen davon gezeigt. Dabei können gegebenenfalls Mehrfachamputationen berücksichtigt werden, indem die einzelnen Anteile addiert werden.

Falls es möglich ist, kann der Faktor k auch individuell über das Wiegen des betreffenden Amputats eines Patienten im Vergleich zu seiner Gesamtmasse ermittelt werden.

Die erfindungsgemäße Vorrichtung geht zur Durchführung des erfindungsgemäßen Verfahrens wie folgt vor: Der Benutzer gibt an der Eingabeeinheit 2 eine Auswahl des Grades der Amputation des Patienten ein. Eine zuvor hinterlegte Angabe kann auch bereits in der Speichereinheit 3 gespeichert sein, so dass eine Neueingabe nicht erforderlich ist. In diesem Fall kann der für den einzelnen Patienten hinterlegte Wert jederzeit über die Eingabeeinheit 2 geändert werden.

Es kann auch sein, dass derartige Angaben über eine Speicherkarte, mit der ein Benutzer den Patienten an der Vorrichtung anmeldet, der Vorrichtung 10 mitgeteilt werden. Hierfür sind dem Fachmann mannigfaltige Lösungen geläufig. Ähnliches gilt für die Übertragung des Computerprogramms in die Mikroprozessorspeichereinheit 1a. Hierzu kann die Eingabeeinheit 2 eine entsprechende Schnittstelle für ein Computerprogrammprodukt, auf dem das Computerprogramm gespeichert ist, aufweisen. Auch hierfür stehen dem Fachmann zahlreiche Möglichkeiten wie CD- oder DVD-Laufwerke, USB-Schnittstellen, serielle wie parallele Bussysteme, Bluetooth- oder Infrarotschnittstellen, Internetanbindung, verschiedene Speicherkartenlesesysteme etc. zur Verfügung.

Die Spannungs- und Stromelektroden werden je nach Amputationsgrad zum Beispiel gemäß den Figuren 2(b)-(d), 3(a) oder 3(b) am Patienten befestigt. Über die Eingabeeinheit 2 wird daraufhin eine Bioimpedanzmessung zum Zeitpunkt t initiiert, bei der ein elektrischer Strom mit einer oder mit multiplen Frequenzen über die Stromelektroden injiziert und entsprechende Spannungswerte gemessen werden. Mit Hilfe der Strom- und Spannungswerte kann die Bioimpedanzmesseinrichtung 5 Körperimpedanzwerte des Patienten ermitteln, die über die Speichereinheit 3 zur der Auswerteeinheit 1 zur weiteren Auswertung zur Verfügung gestellt werden. Die Auswerteeinheit 1 ermittelt daraufhin die Volumenkompartimente des extrazellulären Wassers (ECW), des intrazellulären Wasser (ICW), des Überwässerungsvolumens (OH), des Magergewebes (LTM) und/oder des Fettgewebes (ATM) anhand eines Patientenmodells, das den Amputationsgrad des Patienten zunächst nicht berücksichtigt. Ein solches Verfahren ist zum Beispiel in der bereits zitierten internationalen Patentanmeldung WO 2006/002685 wiedergegeben, auf deren Offenbarung vollumfänglich Bezug genommen wird.

Für die Auswertung der Bioimpedanzmessdaten berücksichtigt die Auswerteeinheit 1 ein korrigiertes Körpergewicht M'(t), das sich aus dem Körpergewicht M(t) geteilt durch den abgespeicherten Korrekturfaktor k ergibt:

$$M'(t) = M(t)/k \qquad (1).$$

Dieses korrigierte Körpergewicht M'(t) wird nun der Ermittlung der Volumenkompartimente auf der Basis der bisherigen Auswertungsprogramme zugrunde gelegt, d.h. die Auswertung erfolgt auf der Basis eines Körpergewichtes des Patienten, als ob ihm keine Gliedmaßen oder Extremitäten fehlen würden. Die nun durch die Auswerteeinheit bestimmten Volumenkompartimente des extrazellulären Wassers (ECW), des intrazellulären Wasser (ICW), des Überwässerungsvolumens (OH), des Magergewebe (LTM) und/oder des Fettgewebes (ATM) werden anschließend in die gewünschten korrigierten Volumenkompartiments V(t) durch Multiplikation mit dem Korrekturfaktor k überführt:

$$ECW(t) = k \cdot ECW'(t) \qquad (2a)$$

$$ICW(t) = k \cdot ICW'(t) \qquad (2b)$$

$$OH(t) = k \cdot OH'(t) \qquad (2c)$$

$$LTM(t) = k \cdot LTM'(t) \qquad (2d)$$

$$ATM(t) = k \cdot ATM'(t) \qquad (2e).$$

Die Ergebnisse werden anschließend an eine Ausgabeeinheit 9 weitergegeben, welche eine Anzeigevorrichtung ist und welche die Ergebnisse dem Benutzer anzeigt. Weitere Ergebnisse - unabhängig davon, ob es sich um Zwischenergebnisse oder Folgeergebnisse handelt - können zu dem informativen Bild auf dem Display hinzugefügt werden.

Die Ergebnisse für die korrigierten Volumenkompartimente können in der Vorrichtung gespeichert werden, um eine Trendanalyse, welche zu einem früheren Zeitpunkt hergeleitete Ergebnisse einschließt, zu ermöglichen. Es hat sich auch als nützlich erwiesen, die Daten durch ein Herleiten gewichteter Durchschnittswerte aus den letzten und den jüngsten Daten zu glätten. Zu diesem Zweck sind verschiedene Algorithmen zur Reduzierung der statistischen Streuung der Daten im Stand der Technik verfügbar. Eine nützliche Verbesserung bei der Durchschnittsberechnung der aktuellen Ergebnisse, die angezeigt werden, wurde durch das Beimessen der höchsten Gewichtung für die letzte Messung und durch ein Herabsetzen der Gewichtung der anderen, früheren Messungen mit zunehmender Zeit, die seit diesen Messungen verstrichen ist, erreicht.

Im Falle der Amputation beider Arme oder beider Beine kann eine Modifikation der Formeln gemäß den Gleichungen (2a)-(2e) notwendig sein. In diesem Fall wird eine Anordnung der Elektroden wie in Fig. 3 gezeigt vorgenommen. Es hat sich dann als sinnvoll erwiesen, die Volumenkompartiments durch eine korrelierte Größe in folgender Form zu korrigieren:

$$ECW(t) = k \cdot \left( ECW'(t) \cdot m_{FF,HH} + o_{FF,HH} \right) \qquad (3a),$$

$$ICW(t) = k \cdot \left( ICW'(t) \cdot m_{FF,HH} + o_{FF,HH} \right) \qquad (3b),$$

$$OH(t) = k \cdot \left( OH'(t) \cdot m_{FF,HH} + o_{FF,HH} \right) \qquad (3c),$$

$$LTM(t) = k \cdot \left( LTM'(t) \cdot m_{FF,HH} + o_{FF,HH} \right) \qquad (3d),$$

$$ATM(t) = k \cdot \left( ATM'(t) \cdot m_{FF,HH} + o_{FF,HH} \right) \qquad (3e).$$

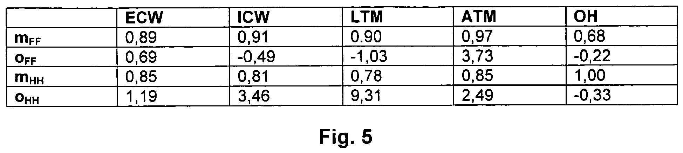

Der Index "FF" steht für den Fall, dass von Fuß zu Fuß (Fig. 3(a)), der Index "HH" dafür, dass von Hand zu Hand gemessen wird (Fig. 3(b)). Beispielhafte Werte für entsprechende Faktoren m und Offsets o sind in Fig. 5 zusammengestellt. Diese Parameter können ebenfalls in der Speichereinheit 3 hinterlegt sein. Im Falle verschiedener Messkonfigurationen kann die erfindungsgemäße Vorrichtung weiter so ausgestaltet sein, dass der Benutzer die Messkonfiguration über die Eingabeeinheit 2 auswählen kann (z.B. Hand zu Fuß, Hand zu Hand oder Fuß zu Fuß), damit die Auswerteeinheit 1 dies bei der Auswertung berücksichtigen kann.

Mit Hilfe der Erfindung gelingt es, Volumenkompartimente auch bei amputierten Patienten in einfacher Weise mit Hilfe von Bioimpandanzmessungen zu bestimmen, wobei auf die bisher für Patienten ohne Amputationen verwendeten und bewährten Körpermodelle ohne größere Adaption zurückgegriffen werden kann. Es ist lediglich die Bestimmung eines für die Amputation charakteristischen Faktors notwendig, der durch Vergleichsmessungen im Vorfeld ermittelt werden kann. In bestimmten Ausnahmesituationen von Mehrfachamputationen kann es angezeigt sein, zur Genauigkeitssteigerung zusätzliche Parameter einfließen zu lassen. Dabei können die bisherigen Körpermodelle auch für die Fälle weiter verwendet werden.

Unter die erfindungsgemäße Lehre und damit auch unter den beanspruchten Schutzbereich fallend werden auch mathematisch gleichwertige Berechnungen verstanden, bei denen Mess-oder Zwischenrechengrößen durch proportionale oder andere eineindeutig zugeordnete Ausdrücke ersetzt werden. Derartige Vorgehensweisen machen von der erfindungsgemäße Lehre gleichermaßen Gebrauch und sollen im Rahmen der verwendeten Terminologie explizit mit einbezogen sein.

## Patentansprüche

1. Verfahren zur Bestimmung eines korrigierten Volumenkompartiments V(t) eines Patienten zum Zeitpunkt t, wobei dem Patienten einzelne Gliedmaßen oder Teile davon fehlen, umfassend die folgenden Schritte:

   Messen von Bioimpedanzdaten am Patienten zum Zeitpunkt t,
   Bestimmen eines Volumenkompartiments V'(t) des Patienten anhand der gemessenen Bioimpedanzdaten,
   Bestimmen eines korrigierten Volumenkompartiments V(t) durch Multiplikation des Volumenkompartiments V'(t) oder einer damit korrelierten Größe mit einem für ein fehlendes Gliedmaß der einzelnen Gliedmaßen oder für Teile davon charakteristischen Korrekturfaktor k,
   **gekennzeichnet durch**:

   Ermitteln des Körpergewichts M(t) des Patienten,
   wobei beim Bestimmen des Volumenkompartiments V'(t) ein korrigiertes Körpergewicht M'(t) verwendet wird, das sich aus dem Quotienten des gemessenen Körpergewichtes M(t) geteilt durch den Korrekturfaktor k ergibt,
   und das Bestimmen des Volumenkompartiments V'(t) anhand der Bioimpedanzdaten und des korrigierten Körpergewichts M'(t) ansonsten so erfolgt, als ob dem Patienten keine Gliedmaßen oder Extremitäten fehlen würden.

2. Verfahren nach Anspruch 1, **dadurch gekennzeichnet, dass** das Volumenkompartiment mindestens eins ausgewählt aus der folgenden Gruppe ist: Magergewebe (LTM), Fettgewebe (ATM), Überwässerungsvolumen (OH), extrazelluläres Wasser (ECW), intrazelluläres Wasser (ICW).

3. Verfahren nach Anspruch 1 oder 2, **dadurch gekennzeichnet, dass** die Bioimpedanzmessung eine Ganzkörpermessung ist.

4. Verfahren nach Anspruch 3, **dadurch gekennzeichnet, dass** die Ganzkörpermessung durch Spannungs- und Stromelektroden durchgeführt wird, die bei Fehlen eines Armes oder Teilen davon und/oder Fehlen eines Beines oder Teilen davon in der Nähe eines vorhandenen Handgelenks und in der Nähe eines vorhandenen Fußgelenks angebracht werden.

5. Verfahren nach Anspruch 3, **dadurch gekennzeichnet, dass** die Ganzkörpermessung durch Spannungs- und Stromelektroden durchgeführt wird, die bei Fehlen beider Beine oder Teilen beider Beine in der Nähe beider Handgelenke angebracht werden.

6. Verfahren nach Anspruch 3, **dadurch gekennzeichnet, dass** die Ganzkörpermessung durch Spannungs- und Stromelektroden durchgeführt wird, die bei Fehlen beider Arme oder Teilen beider Arme in der Nähe beider Fußgelenke angebracht werden.

7. Verfahren nach einem der vorhergehenden Ansprüche, **dadurch gekennzeichnet, dass** der Korrekturfaktor k auf der Basis von Vergleichsdaten ermittelt wird, bei dem der Anteil des fehlenden Gliedmaßes oder des Teiles davon aufgrund seines Anteils am Gewicht eines Vergleichspatienten ohne fehlende Gliedmaße oder Teile davon ermittelt wird.

8. Vorrichtung (10) zur Bestimmung eines korrigierten Volumenkompartiments V(t) eines Patienten zum Zeitpunkt t, wobei dem Patienten einzelne Gliedmaßen oder Teile davon fehlen, mit einer Bioimpedanzmesseinrichtung (5) zur Messung von Bioimpedanzdaten am Patienten, einer Auswerteeinheit (1), die so konfiguriert ist, dass anhand der gemessenen Bioimpedanzdaten ein Volumenkompartiment V'(t) des Patienten bestimmt wird und ein korrigiertes Volumenkompartiment V(t) durch Multiplikation des Volumenkompartiments V'(t) oder einer damit korrelierten Größe mit einem für ein fehlendes Gliedmaß der einzelnen Gliedmaßen oder für Teile davon charakteristischen Korrekturfaktor k bestimmt wird, wobei die Vorrichtung eine Eingabeeinheit (2) zur Eingabe des Körpergewichts M(t) des Patienten aufweist, wobei die Eingabeeinheit (2) ein Eingabegerät zur manuellen Eingabe des Körpergewichtes M(t) des Patienten und/oder eine Waage (7) zur Messung und automatischen Übermittlung des gemessenen Körpergewichtes M(t) des Patienten umfasst, und wobei die Auswerteeinheit (1) **dadurch gekennzeichnet ist, dass** sie so konfiguriert ist, dass sie aufgrund des Körpergewichtes M(t) des Patienten ein korrigiertes Körpergewicht M'(t) bestimmt, das sich aus dem Quotienten des gemessenen Körpergewichtes M(t) geteilt durch den Korrekturfaktor k ergibt, und dass sie das Volumenkompartiment V'(t) mit Hilfe des korrigierten Körpergewichtes M'(t) bestimmt, und wobei das Bestimmen des Volumenkompartiments V'(t) anhand der Bioimpedanzdaten und des korrigierten Körpergewichts M'(t) ansonsten so erfolgt, als ob dem Patienten keine Gliedmaßen oder Extremitäten fehlen würden.

9. Vorrichtung nach Anspruch 8, **dadurch gekennzeichnet, dass** das Volumenkompartiment mindestens eins ausgewählt aus der folgenden Gruppe ist: Magergewebe (LTM), Fettgewebe (ATM), Überwässerungsvolumen (OH), extrazelluläres Wasser (ECW), intrazelluläres Wasser (ICW).

10. Vorrichtung nach Anspruch 8 oder 9, **dadurch gekennzeichnet, dass** die Auswerteeinheit (1) ferner so konfiguriert ist, dass die Bioimpedanzmessung als Ganzkörpermessung ausgewertet wird.

11. Vorrichtung nach Anspruch 10, **dadurch gekennzeichnet, dass** die Auswerteeinheit (1) so konfiguriert ist, dass sie die Ganzkörpermessung auf der Basis auswertet, dass die Spannungs- und Stromelektroden (5a, 5b) bei Fehlen eines Armes oder Teilen davon und/oder Fehlen eines Beines oder Teilen davon in der Nähe eines vorhandenen Handgelenks und in der Nähe eines vorhandenen Fußgelenks angebracht sind.

12. Vorrichtung nach Anspruch 10, **dadurch gekennzeichnet, dass** die Auswerteeinheit (1) so konfiguriert ist, dass sie die Ganzkörpermessung auf der Basis auswertet, dass die Spannungs- und Stromelektroden (5a, 5b) bei Fehlen beider Beine oder Teilen beider Beine in der Nähe beider Handgelenke angebracht sind.

13. Vorrichtung nach Anspruch 10, **dadurch gekennzeichnet, dass** die Auswerteeinheit (1) so konfiguriert ist, dass sie die Ganzkörpermessung auf der Basis auswertet, dass die Spannungs- und Stromelektroden (5a, 5b) bei Fehlen beider Arme oder Teilen beider Arme in der Nähe beider Fußgelenke angebracht sind.

14. Vorrichtung nach einem der Ansprüche 8 bis 13, **dadurch gekennzeichnet, dass** verschiedene Korrekturfaktoren k für unterschiedliche Amputationsgrade in einer Speichereinheit (3) abgespeichert und durch eine Eingabeeinheit (2) auswählbar sind.

15. Computerprogrammprodukt bestehend aus einem Speicherträger mit einem gespeicherten Computerprogramm zur Durchführung des Verfahrens nach einem der Ansprüche 1 bis 7 und/oder zur Konfigurierung der Auswerteeinheit einer der Ansprüche 8 bis 14 in der dort angegebenen Weise durch Übertragung des auf dem Computerprogrammprodukt gespeicherten Computerprogramms in eine Auswerteeinheit einer Vorrichtung, die eine Bioimpedanzmesseinrichtung umfasst.

**Claims**

1. A method for determining a corrected volume compartment V(t) of a patient at the time t, wherein the patient is missing individual limbs or parts thereof, comprising the following steps:

    measuring bioimpedance data on the patient at the time t,
    determining a volume compartment V'(t) of the patient based on the measured bioimpedance data,
    determining a corrected volume compartment V(t) by multiplying the volume compartment V'(t) or a variable correlated therewith with a correction factor k, which is characteristic for a missing limb of the individual limbs or for parts thereof,
    **characterized by**:

    determining the body weight M(t) of the patent, wherein a corrected body weight M'(t), which results from the quotient of the measured body weight M(t), divided by the correction factor k, is used when determining the volume compartment V'(t),
    and the determination of the volume compartment V'(t) based on the bioimpedance data and of the corrected body weight M'(t) otherwise takes place as if the patient were not missing limbs or extremities.

2. The method according to claim 1, **characterized in that** the volume compartment is at least one selected from the following group: lean tissue (LTM), adipose tissue (ATM), overwatering volume (OH), extracellular water (ECW), intracellular water (ICW).

3. The method according to claim 1 or 2, **characterized in that** the bioimpedance measurement is a whole body measurement.

4. The method according to claim 3, **characterized in that** the whole body measurement is carried out by means of voltage and current electrodes, which, when an arm or parts thereof are missing and/or when a leg or parts thereof are missing, are attached in the vicinity of an existing wrist and in the vicinity of an existing ankle.

5. The method according to claim 3, **characterized in that** the whole body measurement is carried out by means of voltage and current electrodes, which, when both legs or parts of both legs are missing, are attached in the vicinity of both wrists.

6. The method according to claim 3, **characterized in that** the whole body measurement tis carried out by means of voltage and current electrodes, which, when both arms or parts of both arms are missing, are attached in the vicinity of both ankles.

7. The method according to one of the preceding claims, **characterized in that** the correction factor k is determined based on comparative data, in the case of which the proportion of missing limb or of the part thereof is determined based on its proportion of the weight of a comparative patient without missing limbs or parts thereof.

8. A device (10) for determining a corrected volume compartment V(t) of a patient at the time t, wherein the patient is missing individual limbs or parts thereof, comprising
    a bioimpedance measuring device (5) for measuring bioimpedance data on the patient,
    an evaluation unit (1), which is configured such that, based on the measured bioimpedance data, a volume compartment V' (t) of the patient is determined and a corrected volume compartment V(t) is determined by multiplying the volume compartment V'(t) or a variable correlated therewith with a correction factor k, which is characteristic for a missing limb of the individual limbs or for parts thereof,
    wherein the device has an input unit (2) for inputting the body weight M(t) of the patient,
    wherein the input unit (2) comprises an input device for manually inputting the body weight M(t) of the patient and/or a scale (7) for measuring an automatic transmission of the measured body weight M(t) of the patient,
    and wherein the evaluation unit (1) is **characterized in that** it is configured such that, based on the body weight M(t) of the patient, it determines a corrected body weight M'(t), which results from the quotient of the measured body weight M(t), divided by the correction factor k, and that it determines the volume compartment V'(t) with the help of the corrected body wight M'(t), and wherein the determination of the volume compartment V'(t) based on the bioimpedance data and the corrected body weight M'(t) otherwise takes place as if the patient were not missing limbs or extremities.

9. The device according to claim 8, **characterized in that** the volume compartment is at least one selected from the following group: lean tissue (LTM), adipose tissue (ATM), overwatering volume (OH), extracellular water (ECW), intracellular water (ICW).

10. The device according to claim 8 or 9, **characterized in that** the evaluation unit (1) is further configured such that the bioimpedance measurement is evaluated as a whole body measurement.

11. The device according to claim 10, **characterized in that** the evaluation unit (1) is configured such that it evaluates the whole body measurement on the basis that the voltage and current electrodes (5a, 5b) are attached in the vicinity of an existing wrist and in the vicinity of an existing ankle when an arm or parts thereof are missing and/or when a leg or parts thereof are missing.

12. The device according to claim 10, **characterized in that** the evaluation unit (1) is configured such that it evaluates the whole body measurement on the basis that the voltage and current electrodes (5a, 5b) are attached in the vicinity of both wrists when both legs or parts of both legs are missing.

13. The device according to claim 10, **characterized in that** the evaluation unit (1) is configured such that it evaluates the whole body measurement on the basis that the voltage and current electrodes (5a, 5b) are attached in the vicinity of both ankles when both arms or parts of both arms are missing.

14. The device according to one of claims 8 to 13, **characterized in that** different correction factors k for different levels of amputation are stored in a memory unit (3) and can be selected by means of an input unit (2).

15. A computer program product consisting of a storage medium comprising a stored computer program for carrying out the method according to one of claims 1 to 7 and/or for configuring the evaluation unit of one of claims 8 to 14 in the manner specified therein by transmitting the computer program stored on the computer program product into an evaluating unit of a device, which comprises a bioimpedance measuring device.


**Revendications**

1. Procédé de définition d'un compartiment volumique corrigé V(t) d'un patient au moment t, dans lequel il manque au patient certaines tronçons de membres ou de parties de ceux-ci, comprenant les étapes suivantes :

   mesure des données de bio-impédance sur le patient au moment t,
   définition d'un compartiment volumique V' (t) du patient à partir des données de bio-impédance mesurées,
   définition d'un compartiment volumique corrigé V(t) par multiplication du compartiment volumique V'(t) ou d'un paramètre corrélatif avec un facteur de correction k caractéristique pour un tronçon de membre manquant parmi les différents tronçons de membres ou pour des parties de ceux-ci,
   **caractérisé par** :

   la détermination du poids corporel M(t) du patient, dans lequel procédé, lors de la définition d'un compartiment volumique V'(t), un poids corporel corrigé M'(t) est utilisé, lequel poids corporel corrigé résulte du quotient du poids corporel mesuré M(t) divisé par le facteur de correction k,
   et la définition du compartiment volumique V' (t) est réalisée autrement à partir des données de bio-impédance et du poids corporel corrigé M'(t) comme s'il ne manquait pas au patient de tronçons de membres ou d'extrémités.

2. Procédé selon la revendication 1, **caractérisé en ce que** le compartiment volumique est au moins un compartiment sélectionné dans le groupe suivant : tissu maigre (LTM), tissu adipeux (ATM), volume de rétention d'eau (OH), eau extracellulaire (ECW), eau intracellulaire (ICW).

3. Procédé selon la revendication 1 ou 2, **caractérisé en ce que** la mesure de bio-impédance est une mesure sur la totalité du corps.

4. Procédé selon la revendication 3, **caractérisé en ce que** la mesure sur la totalité du corps est réalisée par des électrodes à tension et courant qui, en l'absence d'un bras ou de parties de celui-ci et/ou en l'absence d'une jambe ou de parties de celle-ci, sont posées à proximité d'une articulation existante de la main et à proximité d'une articulation existante du pied.

5. Procédé selon la revendication 3, **caractérisé en ce que** la mesure sur la totalité du corps est réalisée par des électrodes à tension et courant qui, en l'absence des deux jambes ou de parties des deux jambes, sont appliquées à proximité des deux articulations des mains.

6. Procédé selon la revendication 3, **caractérisé en ce que** la mesure sur la totalité du corps est réalisée par des électrodes à tension et courant qui, en l'absence des deux bras ou de parties des deux bras, sont appliquées à proximité des deux articulations des pieds.

7. Procédé selon une des revendications précédentes, **caractérisé en ce que** le facteur de correction k est déterminé sur la base de données comparatives et dans lequel la part du tronçon de membre manquant ou de parties de celui-ci est déterminée en partant de sa part par rapport au poids d'un patient comparatif sans tronçons manquants de membres ou de parties de ceux-ci.

8. Dispositif (10) de définition d'un compartiment volumique corrigé V(t) d'un patient au moment t, dans lequel il manque au patient certains tronçons de membres ou parties de ceux-ci, comprenant un dispositif de mesure de bio-impédance (5) pour la mesure de données de bio-impédance sur le patient,
une unité d'exploitation (1) qui est conçue pour, à partir des données de bio-impédance mesurées, définir un compartiment volumique corrigé V(t) par multiplication du compartiment volumique V'(t) ou d'un paramètre corrélatif avec un facteur de correction k caractéristique pour un tronçon de membre manquant ou pour des parties de celui-ci, le dispositif présentant une unité de saisie (2) pour la saisie du poids corporel M(t) du patient,
l'unité de saisie (2) comprenant un appareil de saisie pour la saisie manuelle du poids corporel M(t) du patient et/ou une balance (7) pour la mesure et la transmission automatique du poids corporel mesuré M(t) du patient,
et l'unité d'exploitation (1) étant **caractérisée en ce qu'**elle est conçue pour définir, à partir du poids corporel M(t) du patient, un poids corporel corrigé M'(t) qui résulte du quotient du poids corporel mesuré M(t) divisé par le facteur de correction k, et qu'elle définit le compartiment volumique V' (t) à l'aide du poids corporel corrigé M'(t), la définition du compartiment volumique V' (t) à partir des données de bio-impédance et du poids corporel corrigé M'(t) ayant autrement lieu comme s'il ne manquait pas au patient de tronçons de membres ou d'extrémités.

9. Dispositif selon la revendication 8, **caractérisé en ce que** le compartiment volumique est au moins un compartiment sélectionné dans le groupe suivant : tissu maigre (LTM), tissu adipeux (ATM), volume de rétention d'eau (OH), eau extracellulaire (ECW), eau intracellulaire (ICW).

10. Dispositif selon la revendication 8 ou 9, **caractérisé en ce que** l'unité d'exploitation (1) est en outre conçue pour que la mesure de bio-impédance soit exploitée sous forme d'une mesure de la totalité du corps.

11. Dispositif selon la revendication 10, **caractérisé en ce que** l'unité d'exploitation (1) est conçue pour exploiter la mesure de la totalité du corps sur la base prévoyant que les électrodes à tension et à courant (5a, 5b), en l'absence d'un bras ou de parties de celui-ci et/ou en l'absence d'une jambe ou de parties de celle-ci, soient appliquées à proximité d'une articulation existante de la main et à proximité d'une articulation existante du pied.

12. Dispositif selon la revendication 10, **caractérisé en ce que** l'unité d'exploitation (1) est conçue pour exploiter la mesure de la totalité du corps sur la base prévoyant que les électrodes à tension et à courant (5a, 5b), en l'absence des deux jambes ou de parties de celles-ci, soient appliquées à proximité des deux articulations des mains.

13. Dispositif selon la revendication 10, **caractérisé en ce que** l'unité d'exploitation (1) est conçue pour exploiter la mesure de la totalité du corps sur la base prévoyant que les électrodes à tension et à courant (5a, 5b), en l'absence des deux bras ou de parties de ceux-ci, soient appliquées à proximité des deux articulations des pieds.

14. Dispositif selon une des revendications 8 à 13, **caractérisé en ce que** différents facteurs de correction k pour différent degrés d'amputation peuvent être enregistrés dans une unité de mémoire (3) et sélectionnés par une unité de saisie (2).

15. Produit de programmation informatique composé d'un support de mémoire comportant un programme informatique enregistré pour la réalisation du procédé selon une des revendications 1 à 7 et/ou pour la configuration de l'unité d'exploitation selon une des revendications 8 à 14 de la manière qui y est indiquée par transmission du programme informatique enregistré sur le produit de programmation informatique dans une unité d'exploitation d'un dispositif qui comprend un dispositif de mesure de bio-impédance.

**Fig. 1**

Normale Messung     Amputation Arm     Amputation Bein     Amputation Arm + Bein

(a)       (b)       (c)       (d)

**Fig. 2**

**Amputation beider Arme**
(a)

**Amputation beider Beine**
(b)

## Fig. 3

| Prozent vom Körpergewicht | | Linke Seite | Rechte Seite |
|---|---|---|---|
| ►Kompletter Arm | 6,6% | | |
| ►Oberhalb Ellbogen | 3,1 % | | |
| ►Unterhalb Ellbogen | 1,5 % | | |
| ►Komplettes Bein | 18,5 % | | |
| ►Oberhalb Knie | 8,0 % | | |
| ►Unterhalb Knie | 6,5 % | | |
| **Summe aller Prozente:** | | | |

## Fig. 4

|         | ECW  | ICW   | LTM   | ATM  | OH    |
|---------|------|-------|-------|------|-------|
| $m_{FF}$ | 0,89 | 0,91 | 0.90 | 0,97 | 0,68 |
| $o_{FF}$ | 0,69 | -0,49 | -1,03 | 3,73 | -0,22 |
| $m_{HH}$ | 0,85 | 0,81 | 0,78 | 0,85 | 1,00 |
| $o_{HH}$ | 1,19 | 3,46 | 9,31 | 2,49 | -0,33 |

**Fig. 5**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2006002685 A


**In der Beschreibung aufgeführte Nicht-Patentliteratur**

- **WIESKOTTEN.** Tagungsband Automatisierungstechnische Verfahren für die Medizin. Workshop, 2007

- **ROTTMANN.** Bestimmung der Körperzusammensetzung mittels Bioimpedanzspektroskopie unter Berücksichtigung von Amputationen. *Diplomarbeit,* 14. Mai 2007, 1-69